# EUROPÄISCHE PATENTANMELDUNG

(11) **EP 1 923 465 A1**
(43) Veröffentlichungstag der Anmeldung: **21.05.2008**
(21) Anmeldenummer: 08002377.3
(22) Anmeldetag: 27.10.1998
(51) Int. Cl.: C12N 15/18, C07K 14/475, C07K 16/22, C12N 5/10, A61K 38/22, A61K 48/00, G01N 33/53, C12Q 1/68

(54) **Inhibitor-Protein des WNT-Signalwegs**

(30) Priorität: 27.10.1997 DE 19747418
(62) Teilanmeldung aus: 98959767.9
(71) Anmelder: Deutsches Krebsforschungszentrum, Stiftung des öffentlichen Rechts, 69120 Heidelberg (DE)
(72) Erfinder: Niehrs, Christof, 69117 Heidelberg (DE); Glinka, Andrei, 69126 Heidelberg (DE)
(74) Vertreter: Schüssler, Andrea

(57) **Zusammenfassung**

Die vorliegende Erfindung betrifft ein Inhibitor-Protein des wnt-Signalwegs, eine ein solches Protein kodierende DNA und ein Verfahren zur Herstellung eines solchen Proteins. Ferner betrifft die Erfindung die Verwendung der DNA und des Proteins sowie gegen das Protein gerichtete Antikörper.

SEQUENCE LISTING

<110> Deutsches Krebsforschungszentrum Stiftung des öffentlichen
 Rechts

<120> Inhibitor-Protein des wnt-Signalwegs

<130> K 3315EU

<150> DE 19747418.7
<151> 1997-10-27

<160> 9

<170> PatentIn version 3.2

<210> 1
<211> 1297
<212> DNA
<213> Xenopus laevis

<400> 1
gacagtcgga gccggcgctg cagcatcaaa gggacttatc ttggaggact tgtgaattct 60

catcctgcca ttgtggttac tgagtctggt tggacagagg aatgggcagc aacatgttcc 120

cggtgcctct tattgtcttt tggggtttta tcttggatgg ggcacttggc tttgtcatga 180

tgaccaactc caactccatc aagaatgtgc cggcggcacc agcaggtcag cccattggct 240

actaccctgt gagcgtcagt ccggactccc tatatgatat tgccaacaag taccaacctc 300

tggatgccta cccgctctac agttgcacgg aagatgatga ctgtgccctt gatgaattct 360

gtcacagttc cagaaacggc aactctctgg tttgcttggc atgccggaaa cgcagaaagc 420

gttgcctgag ggacgccatg tgctgcacag gcaactactg tagcaacgga atttgtgtcc 480

ctgtggagca agatcaagag cgcttccaac accagggata cctggaagaa accattctgg 540

aaaactataa taatgctgat catgcaacaa tggatactca ttccaaatta accacgtccc 600

catctggaat gcagcccttt aaaggccgtg atggtgatgt ttgcctccga tcaactgact 660

gtgcgccagg tctatgctgt gcccgtcatt tctggtcaaa gatctgcaag ccggtccttg 720

atgaaggcca agtgtgcacc aagcacagga ggaaaggctc tcacggccta gagattttcc 780

agcgttgtca ctgcggtgcc ggactctcgt gccggttaca gaaaggagaa tttacaactg 840

tccctaaaac atcgagactt cacacttgcc aaagacacta agcgaggcct acagagcctg 900

aaggaccttc tctaaattaa gctaattaag actttggtac ctgcatgtta ttttctcagt 960

ttacatgaag tgctctggtc ttccctgaac ccggaagctg cgcaacttgt ttcttttttt 1020

gaggaacttc ctaattaatg ctaattacag taaattactg tgttgtaaat actacgcaag 1080

gagacctgta aaaactgtaa atacccgtgt atagaaagtg tacatgatct tctctattgt 1140

aacctgccac cttgtacatt ccgacgcgct cttccctttt tatatatata tatatataaa 1200

tatatattat attatgtaga gtttacgtct agtatgtctg tatttttaat tgaaataaaa 1260

catttctaaa cttaaaaaca aaaaaaaaaa aaaaaaa 1297

<210> 2
<211> 881
<212> DNA
<213> Mus musculus

<400> 2
tgcaggcatg aacaaggact gggttcggcg gcagtgagaa gggcaaaagc ctggggcagg 60

cctacccttg cagcagtgat aaggaatgtg aagttggaag atactgccac agtccccacc 120

aaggttcatc agcctgcatg ctctgtagga ggaaaaagaa acgatgccac agagatggga 180

tgtgttgccc tggtacccgc tgcaataatg gaatctgcat cccagtcact gagagcatcc 240

tcaccccaca tatcccagct ctggatggca cccggcatag agatcgcaac catggtcact 300

attccaacca tgacctggga tggcagaatc taggaaggcc acactccaag atgcctcata 360

taaaaggaca tgaaggagac ccatgcctac ggtcatcaga ctgcattgat gggttttgtt 420

gtgctcgcca cttctggacc aaaatctgca aaccagtgct ccatcagggg gaagtctgta 480

ccaaacaacg caagaagggt tcgcacgggc tggagatttt ccagaggtgt gactgtgcaa 540

agggcctgtc ctgcaaagtg tggaaagatg ccacctactc ttccaaagcc agactccatg 600

tatgccagaa gatctgataa acactggaag agtcatcact agcagactgt gaatttgtgt 660

atttaatgca ttatggcatg atggaaacct ggattggaat gcggaagaat gagggatgtg 720

gtaagaatgt ggagcagaag agggcaggac tgaatcaagt agagtcgaca acaaccaaag 780

tactaccagt gcttccgtta tgtgcctcat ctatgtaaat aatgtacaca tttgtgaaaa 840

tgctattatt aaaagaaagc acaccatgga aattacaaaa a 881

<210> 3
<211> 1226
<212> DNA
<213> Mus musculus

<400> 3
gacccacgcg tccgtgcctg tttgcgtcct tcggagatga tggttgtgtg tgcaccggca 60

gctgtccggt tcttggccgt gtttacaatg atggctctct gcagcctccc tctgctagga 120

gccagtgcca ccttgaactc agttctcatc aattccaacg cgatcaagaa cctgccccca 180

ccgctgggtg gtgctggggg gcagccgggc tctgctgtca gtgtggcgcc gggagttctc 240

tatgagggcg ggaacaagta ccagactctt gacaactacc agccctaccc ttgcgctgaa 300

gatgaggagt gcggctctga cgagtactgc tccagcccca gccgcggggc agccggcgtc 360

ggaggtgtac agatctgtct ggcttgccga aagcgcagga agcgctgcat gacgcacgct 420

atgtgctgcc ccgggaacta ctgcaaaaat ggaatatgca tgccctctga ccacagccat 480

tttcctcgag gggaaattga ggaaagcatc attgaaaacc ttggtaatga ccacaacgcc 540

gccgcggggg atggatatcc cagaagaacc acactgactt caaaaatata tcacaccaaa 600

ggacaagaag gctccgtctg cctccgatca tcagactgtg ccgcagggct gtgttgtgca 660

agacacttct ggtccaagat ctgtaaacct gtccttaaag aaggtcaggt gtgcaccaag 720

cacaaacgga aaggctccca cgggctggag atattccagc gctgttactg cggggaaggc 780

ctggcttgca ggatacagaa agatcaccat caagccagca attcttctag gctccacacc 840

tgccagagac actaaaccga cagtctaaat atgatggact ctttttatct aatatatgct 900

acgaaaatcc tttatgattt gtcagctcaa tcccaaggat gtaggaatct tcagtgtgta 960

attaagcatt ccgacaatac tttccaaaag ctctggagtg taaggacttt gtttcttgat 1020

ggaactcccc tgtgattgca gtaaattact gtgttgtaaa tcctcagtgt ggcacttacc 1080

tgtaaatgca gcaaaacttt taattatttt tctagaggtg tggtacattg ccttgtttct 1140

cttgcatgta aatttttttt gtacacggtt gattgtcttg actcataaat attctatatt 1200

ggagtagaaa aaaaaaaaa aaaaaa 1226

<210> 4
<211> 768
<212> DNA
<213> Homo sapiens

<400> 4
atacgactca ctatagggaa tttggccctc gaggccaaga attcggcacg agggttggga 60

ggtattgcca cagtccccac caaggatcat cggcctgcat ggtgtgtcgg agaaaaaaga 120

agcgctgcca ccgagatggc atgtgctgcc ccagtacccg ctgcaataat ggcatctgta 180

tcccagttac tgaaagcatc ttaacccctc acatcccggc tctggatggt actcggcaca 240

gagatcgaaa ccacggtcat tactcaaacc atgacttggg atggcagaat ctaggaagac 300

cacacactaa gatgtcacat ataaaagggc atgaaggaga cccctgccta cgatcatcag 360

actgcattga agggttttgc tgtgctcgtc atttctggac caaaatctgc aaaccagtgc 420

tccatcaggg ggaagtctgt accaaacaac gcaagaaggg ttctcatggg ctggaaattt 480

tccagcgttg cgactgtgcg aagggcctgt cttgcaaagt atggaaagat gccacctact 540

cctccaaagc cagactccat gtgtgtcaga aaatttgatc accattgagg aacatcatca 600

attgcagact gtgaagttgt gtatttaatg cattatagca tggtggaaaa taaggttcag 660

atgcagaaga atggctaaaa taagaaacgt gataagaata tagatgatca caaaaaaaaa 720

aaaaaaaaag atgcggccgc aagcttattc cctttagtga gggttaat 768

<210> 5
<211> 828
<212> DNA
<213> Homo sapiens

<400> 5
tggccccgca cgccaaaaat tcggcacgag ggtctggcac tcagaggatg ctctgacctt 60

gaaagggtcc tatctggaga cgagggagta caacgtgctg aatgtgtgcg gttcagggag 120

catttggtaa ccctgcattt gggagcagtg ggcactaacc ggttttggag aggtggacac 180

ataaggactg tgatcagcgc ccgggtccaa gagggcgggt acctggacct ctgggtgcct 240

caccctctcc ccgaaccctt cccacagccg tacccgtgcg cagaggacga ggagtgcggc 300

actgatgagt actgcgctag tcccaccccg cggaggggac cgccggccgt gcaaatctgt 360

ctcgcctgca ggaagcgccg aaaacgctgc atgcgtcacg ctatgtgctg ccccgggaat 420

tactgcaaaa atggaatatg tgtgtcttct gatcaaaatc atttccgagg agaaattgag 480

gaaaccatca ctgaaagctt tggtaatgat catagcacct tggatgggta ttccagaaga 540

accaccttgt cttcaaaaat gtatcacacc aaaggacaag aaggttctgt ttgtctccgg 600

tcatcagact gtgcctcagg attgtgttgt gctagacact tctggtccaa gatctgtaaa 660

cctgtcctga aagaaggtca agtgtgtacc aagcatagga gaaaaggctc tcatggacta 720

gaaatattcc agcgttgtta ctgtggagaa ggtctgtctt gccggataca gaaagatcac 780

catcaagcca gtaattcttc taggcttcac acttgtcaga gacactaa 828

<210> 6
<211> 432
<212> DNA
<213> Homo sapiens

<400> 6
gcggtggcgg ccgctctaga atagtggatc ccccgggctg caggaattcg gcacgagcgg 60

ctgcgggcgc agagcggaga tgcagcggct tgggccaccc tgctgtgcct gctgctggcg 120

gcggcggtcc ccacggcccc cgcgcccgct ccgacggcga cctcggctcc agtcaagccc 180

ggcccggctc tcagctaccc gcaggaggag gccaccctca atgagatgtt ccgcgggtga 240

ggaactgatg gaggacacgc agcacaaatt gcgcagcgcg gtggaagaga tggaggcaga 300

agaagctgct gctaaagcaa tcatcagaag tgaacctggc aaacttacct cccagctatc 360

acaatgagac caacacagac acgaaggttg gaaataataa ccatccatgt gcaccgagaa 420

attcacaagt tt                                                          432

<210>  7
<211>  1383
<212>  DNA
<213>  Gallus gallus

<400>  7
cggcgagcgg cagcggcggc tgaggagcgc cggggatgcg gcggggagag ggaccggcgc      60

cgcggcggcg atggctgctg ctgttggccg tgctggcggc tctgtgctgc gccgcggccg     120

ggagcggcgg gcggcggcga gcggccagcc tgggcgagat gctgcgggag gtggaggcgc     180

tgatggagga cacgcagcac aagctgcgca acgccgtgca ggagatggaa gctgaagaag     240

aaggggcaaa aaaactgtca gaagtaaact ttgaaaactt acctcccacc taccataatg     300

agtccaacac agaaaccaga attggtaata aaactgttca gactcatcaa gaaattgata     360

aggttacaga taacagaact ggatcaacaa ttttttccga gacaattatt acatctataa     420

agggtggaga aaacaaaaga aatcatgagt gtatcattga tgaagactgt gaaacaggaa     480

agtattgcca gttctccacc tttgaataca agtgtcagcc ctgtaaaacc cagcatacac     540

actgctcacg agatgttgaa tgctgcggag accagctttg tgtttggggt gagtgcagga     600

aagccacttc aagaggagaa aatggtacca tttgtgagaa ccaacatgac tgcaacccag     660

gaacgtgctg tgcttttcag aaagaactgc tgtttcctgt gtgcactccg ttacccgaag     720

aaggtgaacc ttgccatgat ccttcaaaca gacttctcaa cctgatcacc tgggaactgg     780

aacctgatgg agtactagag cgctgcccat gtgcaagtgg cttgatctgc caacctcaga     840

gcagccacag tactacatct gtgtgtgaac tgtcctccaa tgaaaccagg aaaaacgaaa     900

aagaagatcc cttgaacatg gatgagatgc catttatcag tttaataccc agagatattc     960

tttctgatta cgaagaaagc agcgtcattc aggaagtgcg taaagaatta gaaagcctgg    1020

aggaccaagc aggtgtgaag tctgagcatg acccggctca tgacctattt ctgggagatg    1080

aaatatgaag ttcaaacacc agtttagtta gtcctagaaa ttgttgtcta gtgtcttgct    1140

tacatacacc cttaacagat actgctggat agaagtgcaa taaacatctt cattgagcat    1200

ccgttttcgt gcaccaaacc tgcatgttca aattcatgtt gaattcactc aatctttgga    1260

ccaaactttc catcaaagac aaatgagaaa ggcatcagtg tttcctttgg attaatcctt    1320

tcctttgtac agcagaaata aacgtatcag tactcgtact cattaaaaaa acacacggag    1380

cat                                                                  1383

<210>  8

<211>  44
<212>  PRT
<213>  artificial sequence

<220>
<223>  wnt consensus sequence


<220>
<221>  misc_feature
<222>  (2)..(16)
<223>  Xaa can be any naturally occurring amino acid

<220>
<221>  misc_feature
<222>  (18)..(19)
<223>  Xaa can be any naturally occurring amino acid

<220>
<221>  misc_feature
<222>  (21)..(26)
<223>  Xaa can be any naturally occurring amino acid

<220>
<221>  misc_feature
<222>  (28)..(32)
<223>  Xaa can be any naturally occurring amino acid

<220>
<221>  misc_feature
<222>  (35)..(38)
<223>  Xaa can be any naturally occurring amino acid

<220>
<221>  misc_feature
<222>  (40)..(41)
<223>  Xaa can be any naturally occurring amino acid

<220>
<221>  misc_feature
<222>  (43)..(43)
<223>  Xaa can be any naturally occurring amino acid

<400>  8

Cys Xaa Xaa Xaa Xaa Xaa Xaa Xaa Xaa Xaa Xaa Xaa Xaa Xaa Xaa Xaa
1               5                   10                  15


Cys Xaa Xaa Cys Xaa Xaa Xaa Xaa Xaa Xaa Cys Xaa Xaa Xaa Xaa Xaa
            20                  25                  30


Cys Cys Xaa Xaa Xaa Xaa Cys Xaa Xaa Gly Xaa Cys
        35                  40


<210>  9
<211>  64
<212>  PRT

<213>  artificial sequence

<220>
<223>  wnt consensus sequence


<220>
<221>  misc_feature
<222>  (2)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(11)
<223>  Xaa can be any naturally occurring amino acid

<220>
<221>  misc_feature
<222>  (14)..(14)
<223>  Xaa can be any naturally occurring amino acid

<220>
<221>  misc_feature
<222>  (16)..(16)
<223>  Xaa can be any naturally occurring amino acid

<220>
<221>  misc_feature
<222>  (20)..(28)
<223>  Xaa can be any naturally occurring amino acid

<220>
<221>  misc_feature
<222>  (30)..(30)
<223>  Xaa can be any naturally occurring amino acid

<220>
<221>  misc_feature
<222>  (32)..(35)
<223>  Xaa can be any naturally occurring amino acid

<220>
<221>  misc_feature
<222>  (37)..(38)
<223>  Xaa can be any naturally occurring amino acid

<220>
<221>  misc_feature
<222>  (40)..(54)
<223>  Xaa can be any naturally occurring amino acid

<220>
<221>  misc_feature
<222>  (57)..(57)
<223>  Xaa can be any naturally occurring amino acid

<220>
<221>  misc_feature
<222>  (59)..(60)
<223>  Xaa can be any naturally occurring amino acid

<220>
<221>  misc_feature
<222>  (63)..(63)
<223>  Xaa can be any naturally occurring amino acid

<400>  9

Gly Xaa Xaa Gly Xaa Xaa Cys Xaa Xaa Xaa Xaa Asp Cys Xaa Gly Xaa
1               5                   10                  15

Cys Cys Ala Xaa Xaa Xaa Xaa Xaa Xaa Xaa Xaa Xaa Cys Xaa Pro Xaa
            20                  25                  30

Xaa Xaa Xaa Gly Xaa Xaa Cys Xaa Xaa Xaa Xaa Xaa Xaa Xaa Xaa Xaa
        35                  40                  45

Xaa Xaa Xaa Xaa Xaa Xaa Arg Cys Xaa Cys Xaa Xaa Gly Leu Xaa Cys
    50                  55                  60

## Beschreibung

Die vorliegende Erfindung betrifft ein Inhibitor-Protein des wnt-Signalwegs, eine ein solches Protein kodierende DNA und ein Verfahren zur Herstellung eines solchen Proteins. Ferner betrifft die Erfindung die Verwendung der DNA und des Proteins sowie gegen das Protein gerichtete Antikörper.

Der wnt-Signalweg spielt eine wichtige Rolle in der Regulation der Zellproliferation und -Differenzierung während der Embryonal-Entwicklung von Drosophila, Xenopus laevis und der Maus. Der wnt-Signaiweg umfaßt die Kombination von sekretorischen Glykoproteinen, die durch wnt-Gene, z.B. Xwnt-8, kodiert sind, und wnt-Rezeptoren, an die die Glykoproteine binden. Ferner ist der wnt-Signalweg beim Menschen kausal im Colon- und Mammakarzinom sowie dem Melanom impliziert (vgl. Peifer, M., Science 275, (1997), 1752-1753). Inhibitoren des wnt-Signalwegs könnten daher eine Möglichkeit darstellen, therapeutisch bei Tumorerkrankungen eingreifen zu können.

Der vorliegenden Erfindung liegt somit die Aufgabe zugrunde, ein Mittel bereitzustellen, mit dem der wnt-Signalweg inhibiert werden kann.

Erfindungsgemäß wird dies durch die Gegenstände in den Patentansprüchen erreicht.

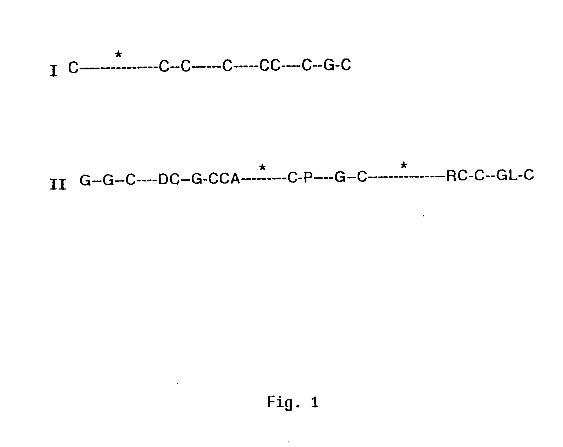

Gegenstand der vorliegenden Erfindung ist somit ein Inhibitor-Protein des wnt-Signalwegs, wobei das Protein zumindest eine der in Fig. 1 angegebenen Aminosäure-Konsensus-Sequenzen I und II umfaßt.

Die vorliegende Erfindung beruht auf der Erkenntnis des Anmelders, daß in Tieren, besonders Säugetieren, ganz besonders dem Menschen, ein Protein existiert, das den wnt-Signalweg inhibiert. Der Anmelder hat gefunden, daß die Expression des wnt-Gens, Xwnt-8, in Xenopus laevis zur Ausbildung von Siamesischen Zwillingen führt. Diese Mißbildung wird verhindert, wenn gleichzeitig das vorstehende Protein exprimiert wird. Dieses Protein ist ein sekretorisches Protein von etwa 40 kD. Es weist zumindest eine der in Fig. 1 angegebenen Cystein-reichen Aminosäure-Konsensus-Sequenzen I und II auf. Varianten des Proteins sind in Form ihrer DNAs in Fig. 2 angegeben. Desweiteren hat der Anmelder erkannt, daß Varianten des Proteins in unterschiedlichen Geweben exprimiert werden (vgl. Tabelle 1 und Fig. 3).

In der vorliegenden Erfindung wird vorstehendes Protein mit "wnt-Inhibitor" (wnt-1) bezeichnet.

In bevorzugter Ausführungsform weist (wnt-1) die in Fig. 1 angegebenen Aminosäure-Konsenus-Sequenzen I und II auf.

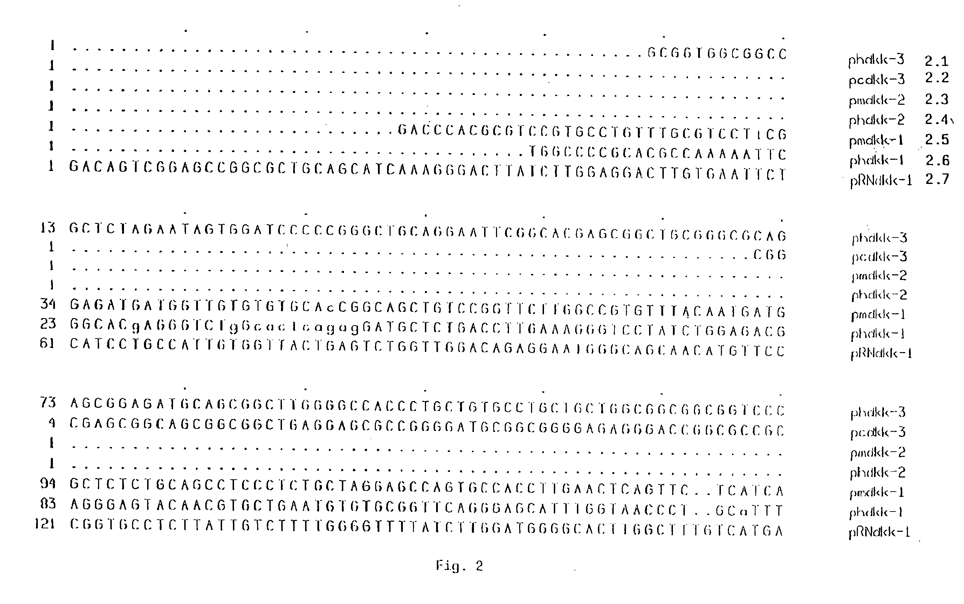

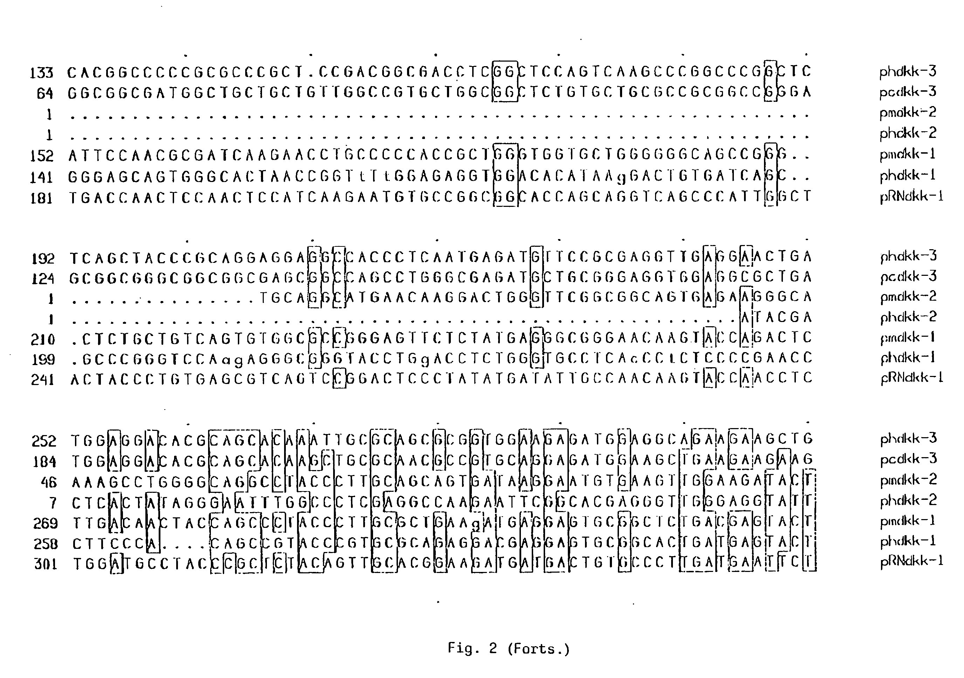

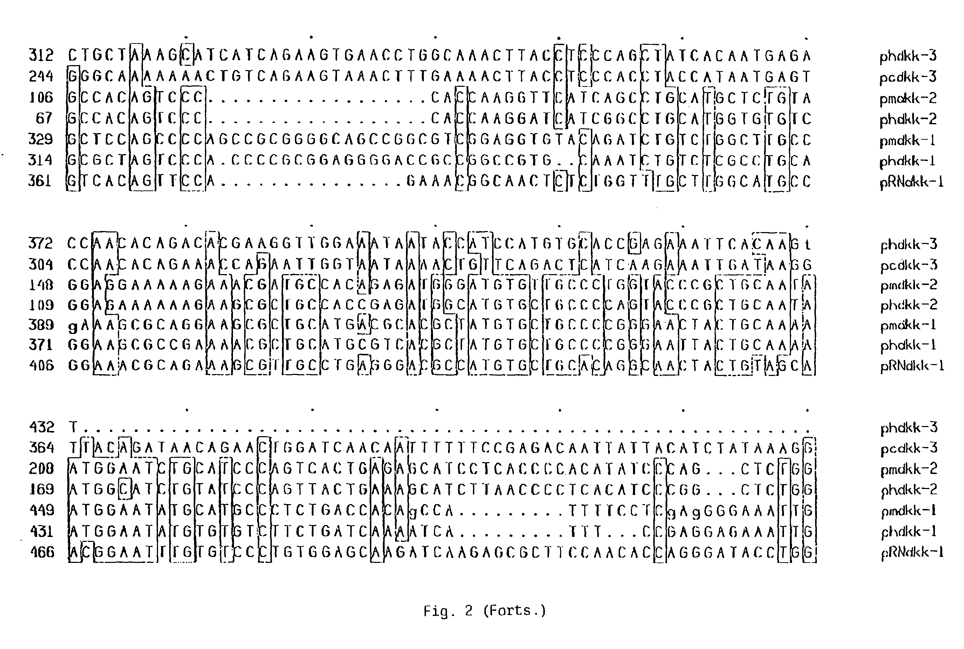

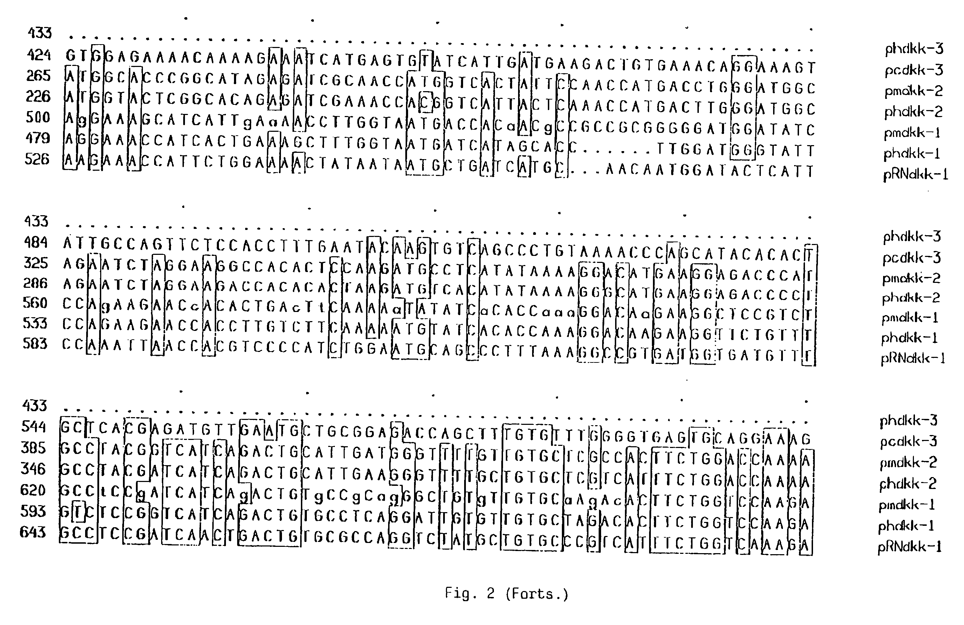

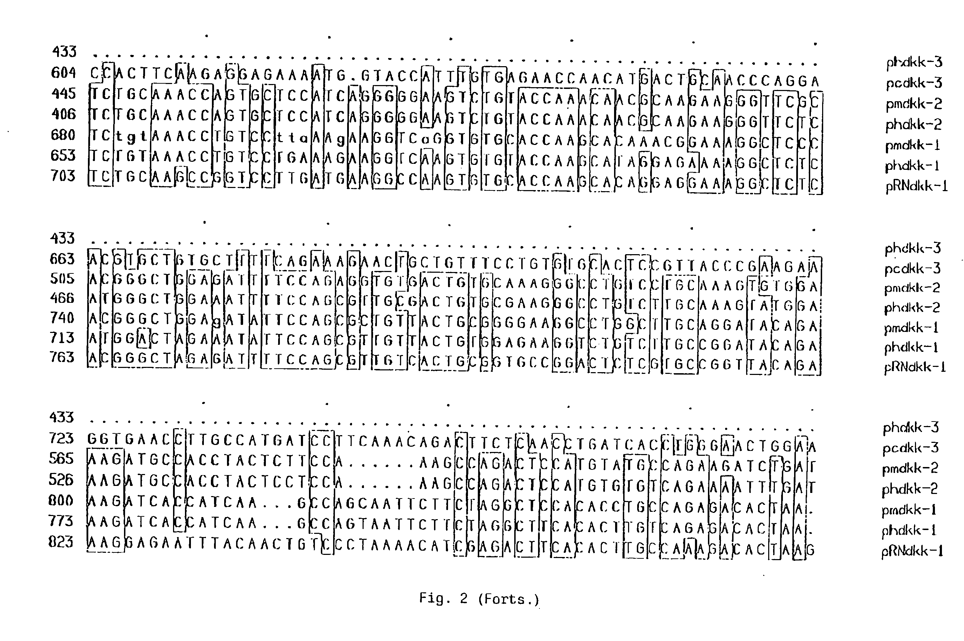

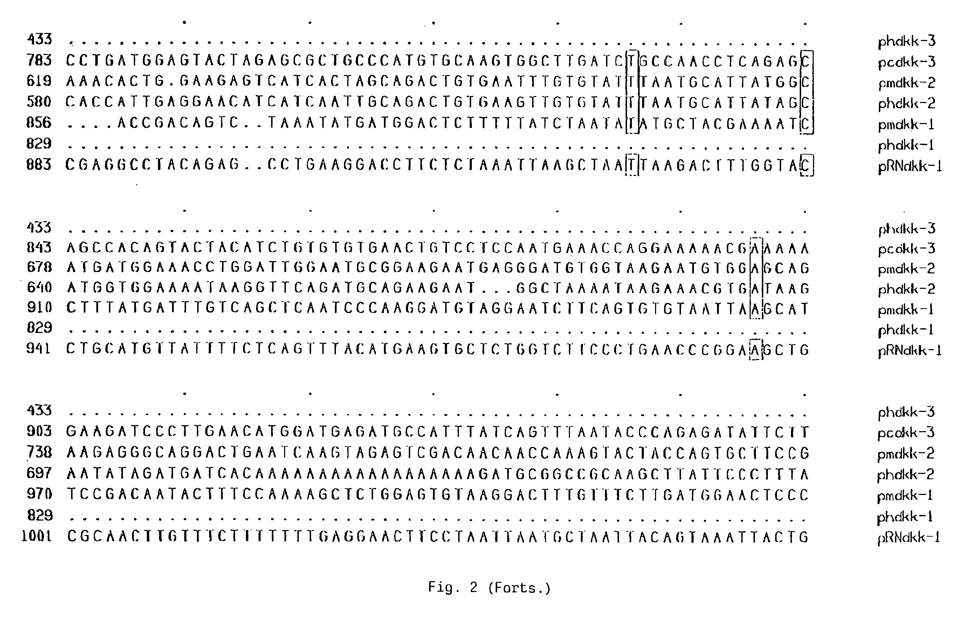

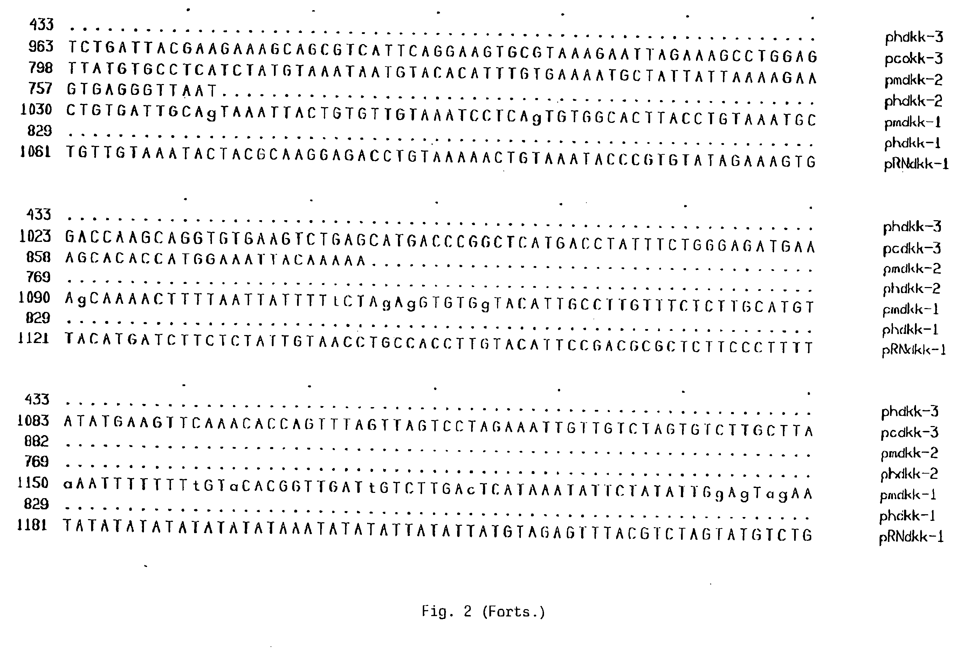

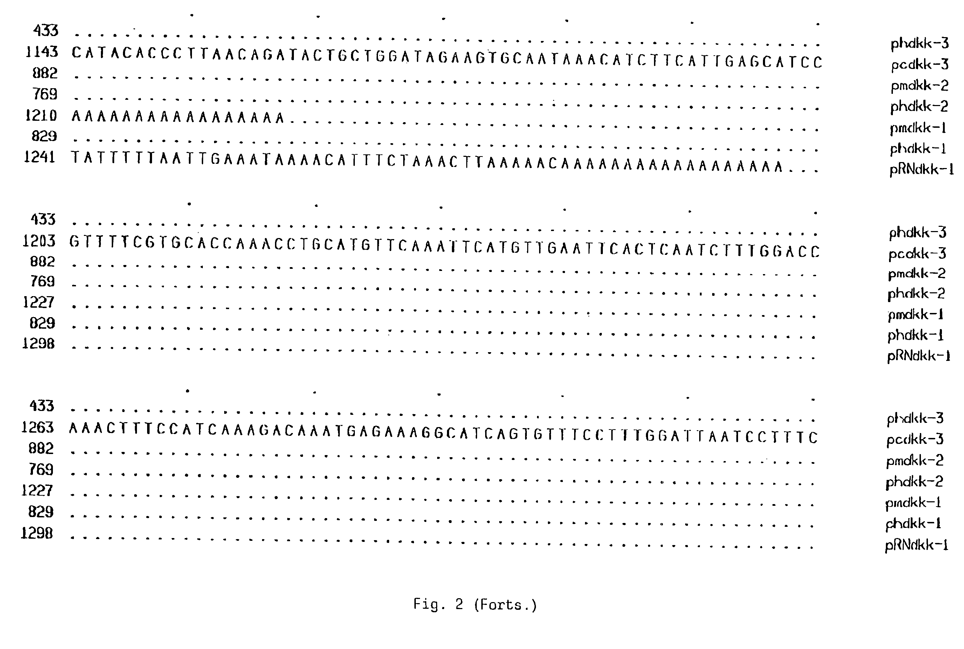

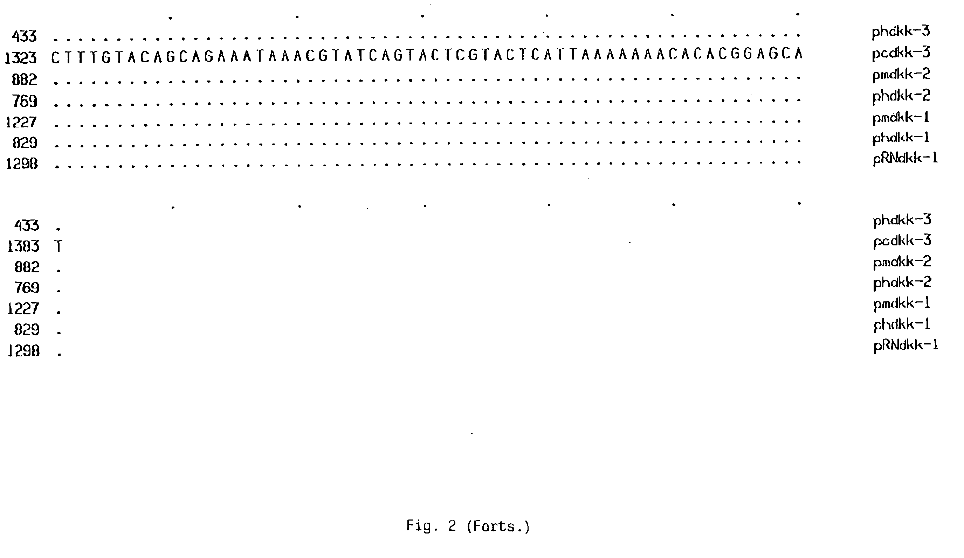

Ein weiterer Gegenstand der vorliegenden Erfindung ist eine für (wnt-I) kodierende Nukleinsäure. Diese kann eine RNA oder eine DNA sein. Letztere kann z.B. eine genomische DNA oder eine cDNA sein. Bevorzugt ist eine DNA, die folgendes umfaßt:
(a) die DNA von Fig. 2 oder eine hiervon durch ein oder mehrere Basenpaare unterschiedliche DNA,
(b) eine mit der DNA von (a) hybridisierende DNA, oder
(c) eine mit der DNA von (a) oder (b) über den degenerierten genetischen Code verwandte DNA.

Der Ausdruck "hybridisierende DNA" weist auf eine DNA hin, die unter üblichen Bedingungen, insbesondere bei 20°C unter dem Schmelzpunkt der DNA, mit einer DNA von (a) hybridisiert.

Die DNA von Fig. 2 umfaßt sieben DNAs, die aus Xenopus laevis, Maus, Mensch oder Huhn stammen und für (wnt-1) kodieren. Sechs dieser DNAs wurden bei der DSMZ (Deutsche Sammlung von Mikroorganismen und Zellkulturen) am 19. Sept. 1997 wie folgt hinterlegt:
- Fig. 2.1: (DNA aus Mensch) als phdkk-3 unter DSM 11762
- Fig. 2.2: (DNA aus Huhn) trägt die Bezeichnung pcdkk-3
- Fig. 2.3: (DNA aus Maus) als pmdkk-2 unter DSM 11759
- Fig. 2.4: (DNA aus Mensch) als phdkk-2 unter DSM 11761
- Fig. 2.5: (DNA aus Maus) als pmdkk-1 unter DMS 11758
- Fig. 2.6: (DNA aus Mensch) als phdkk-1 unter DSM 11760
- Fig. 2.7: (DNA aus Xenopus laevis) als pRNdkk-1 unter DSM 11757

Nachstehend wird eine erfindungsgemäße DNA in Form einer cDNA beschrieben. Diese steht beispielhaft für jede unter die vorliegende Erfindung fallende DNA.

Zur Herstellung einer erfindungsgemäßen cDNA ist es günstig, von einer Xenopus laevis-cDNA-Bibliothek auszugehen (vgl. Glinka, A. et al., Mechanisms Develope 60, (1996), 221-231). Von den einzelnen cDNA-Klonen werden mittels RNA-Polymerase entsprechende mRNAs synthetisiert. Diese werden zusammen mit mRNA von wnt-Genen, z.B. Xwnt-8, in Xenopus laevis mikroinjiziert. Es wird auf die Ausbildung von Siamesischen Zwillingen bei Xenopus laevis gescreent. Diese werden erhalten, wenn die mRNA des wnt-Gens alleine oder zusammen mit solcher Xenopus laevis mRNA mikroinjiziert wird, die nicht für (wnt-I) kodiert. Das Nicht-Auftreten von Siamesischen Zwillingen wird somit als Nachweis für das Vorliegen einer mRNA gewertet, die für (wnt-I) kodiert. Solch eine mRNA läßt unmittelbar die entsprechende cDNA erkennen.

Eine erfindunggemäße cDNA kann in einem Vektor bzw. Expressionsvektor vorliegen. Beispiele solcher sind dem Fachmann bekannt. Im Falle eines Expressionsvektors für E. coli sind dies z.B. pGEMEX, pUC-Derivate, pGEX-2T, pET3b und pQE-8. Für die Expression in Hefe sind z.B. pY100 und Ycpad1 zu nennen, während für die Expression in tierischen Zellen z.B. pKCR, pEFBOS, cDM8 und pCEV4, anzugeben sind. Für die Expression in Insektenzellen eignet sich besonders der Baculovirus-Expressionsvektor pAcSGHisNT-A.

Der Fachmann kennt geeignete Zellen, um eine, erfindungsgemäße, in einem Expressionsvektor vorliegende cDNA zu exprimieren. Beispiele solcher Zellen umfassen die E.coli-Stämme HB101, DH1, x1776, JM101, JM 109, BL21 und SG 13009, den Hefe-Stamm Saccharomyces cerevisiae und die tierischen Zellen L, 3T3, FM3A, CHO, COS, Vero und HeLa sowie die Insektenzellen sf9.

Der Fachmann weiß, in welcher Weise eine erfindungsgemäße cDNA in einen Expressionsvektor inseriert werden muß. Ihm ist auch bekannt, daß diese cDNA in Verbindung mit einer für ein anderes Protein bzw. Peptid kodierenden DNA inseriert werden kann, so daß die erfindungsgemäße cDNA in Form eines Fusionsproteins exprimiert werden kann.

Des weiteren kennt der Fachmann Bedingungen, transformierte bzw. transfizierte Zellen zu kultivieren. Auch sind ihm Verfahren bekannt, das durch die erfindungsgemäße cDNA exprimierte Protein zu isolieren und zu reinigen. Ein solches Protein, das auch ein Fusionsprotein sein kann, ist somit ebenfalls Gegenstand der vorliegenden Erfindung.

Ein weiterer Gegenstand der vorliegenden Erfindung ist ein gegen ein vorstehendes Protein bzw. Fusionsprotein gerichteter Antikörper. Ein solcher Antikörper kann durch übliche Verfahren hergestellt werden. Er kann polyklonal bzw. monoklonal sein. Zu seiner Herstellung ist es günstig, Tiere, insbesondere Kaninchen oder Hühner für einen polyklonalen und Mäuse für einen monoklonalen Antikörper, mit einem vorstehenden (Fusions)protein oder Fragmenten davon zu immunisieren. Weitere "Booster" der Tiere können mit dem gleichen (Fusions)protein oder Fragmenten davon erfolgen. Der polyklonale Antikörper kann dann aus dem Serum bzw. Eigelb der Tiere erhalten werden. Für den monoklonalen Antikörper werden Milzzellen der Tiere mit Myelomzellen fusioniert.

Die vorliegende Erfindung ermöglicht es, den wnt-Signalweg besser zu untersuchen und zu verstehen. Mit einem erfindungsgemäßen Antikörper kann (wnt-1) in Organismen nachgewiesen werden. Ferner kann mit einem erfindungsgemäßen (wnt-I) ein gegen dieses Protein gerichteter Autoantikörper nachgewiesen werden. Beide Nachweise können durch übliche Verfahren, insbesondere einen Western Blot, einen ELISA, eine Immunpräzipitation oder durch Immunfluoreszenz, erfolgen. Desweiteren kann mit einer erfindungsgemäßen Nukleinsäure, insbesondere einer DNA und hiervon abgeleiteten Primern, die Expression des für (wnt-I) kodierenden Gens nachgewiesen werden. Dieser Nachweis kann in üblicher Weise, insbesondere in einem Southern Blot, erfolgen.

Somit können mit der vorliegenden Erfindung auch Prozesse besser untersucht, d.h. diagnostiziert, und verstanden werden, die mit dem wnt-Signalweg zusammenhängen. Dies sind z.B. Zellproliferation und -Differenzierung sowie Erkrankungen verschiedenster Art. Beispiele von letzteren sind Erkrankungen des Auges und der Knochen sowie Tumorerkrankungen, insbesondere Colon- und Mammakarzinom sowie Melanom.

Desweiteren eignet sich die vorliegende Erfindung, Maßnahmen für und gegen das Vorliegen von (wnt-I) in Organismen zu ergreifen. Mit einem erfindungsgemäßen Antikörper kann (wnt-I) in Organismen inhibiert werden. Andererseits kann mit einem erfindungsgemäßen (wnt-I), insbesondere nach Kopplung an ein vom Körper nicht als fremd angesehenes Protein, z.B. Transferrin oder BSA, die Menge von (wnt-I) in Organismen erhöht werden. Entsprechendes kann auch mit einer erfindungsgemäßen Nukleinsäure, insbesondere einer DNA, erreicht werden, die unter die Kontrolle eines in bestimmten Geweben induzierbaren Promotors gestellt wird und nach ihrer Expression zur Bereitstellung von (wnt-I) in diesen Geweben führt. Darüberhinaus kann eine erfindungsgemäße Nukleinsäure, insbesondere eine DNA, auch zur Inhibierung von (wnt-I) genutzt werden. Hierzu wird die Nukleinsäure, z.B. als Basis für die Erstellung von Anti-Sinn-Oligonukleotiden zur Expressions-Inhibierung des für (wnt-I) kodierenden Gens verwendet.
Somit stellt die vorliegende Erfindung auch die Möglichkeit bereit, in den wnt-Signalweg aktivierend bzw. inhibierend einzugreifen. Erstes könnte z.B durch Verabreichung eines erfindungsgemäßen Antikörpers gegen (wnt-I) erfolgen. Für letzteres bietet sich an, erfindungsgemäßes (wnt-I) zu verabreichen. Die Aktivierung des wnt-Signalwegs könnte sinnvoll sein, wenn daran gedacht wird, Organismen für Organspende zu züchten. Die Inhibierung des wnt-Signalwegs bietet sich allerdings an, um therapeutisch bei Erkrankungen von Knochen und des Auges sowie bei Tumorerkrankungen, insbesondere Colon- und Mammakarzinomen sowie Melanom, eingreifen zu können.

Insbesondere zeichnet sich die vorliegende Erfindung dadurch aus, daß sie gewebespezifisch eingesetzt werden kann. Dies gilt sowohl für Diagnose als auch für Therapie. Beispieisweise eignet sich eine erfindungsgemäße DNA, DKK-1, ein entsprechendes Protein bzw. ein Antikörper davon besonders für Gewebe, wie Gehirn, Herz, Gefäße, Knochen, Knorpel, Bindegewebe und Auge. Ferner eignet sich eine erfindungsgemäße DNA, DKK-2, ein entsprechendes Protein bzw. ein Antikörper davon besonders für Gewebe, wie Gehirn, Herz, Gefäße, Knochen, Bindegewebe, Nieren, Hoden, Milz, Ovarien, Muskel, Uterus, Knorpel, Auge und Brustdrüse. Desweiteren eignet sich eine erfindungsgemäße DNA, DKK-3, ein entsprechendes Protein bzw. ein Antikörper davon, besonders für Gewebe, wie Gehirn, Herz, Gefäße, Knochen, Knorpel, Auge, Bindegewebe, Lunge, Ovarien, Muskel und Brustdrüse.

### Kurze Beschreibung der Zeichnungen:

- Fig. 1: zeigt die Aminosäure-Konsensus-Sequenzen I und II eines erfindungsgemäßen (wnt-I). Die Angabe "-" bedeutet eine Aminosäure, wobei die Zahl der Aminosäuren variabel ist, wenn sie einen Stern aufweisen,
- Fig. 2: zeigt die Basensequenz von sieben (wnt-I) kodierenden DNAs mit Angabe der Basen, die zu den Aminosäure-Konsensus-Sequenzen von (wnt-I) beitragen.

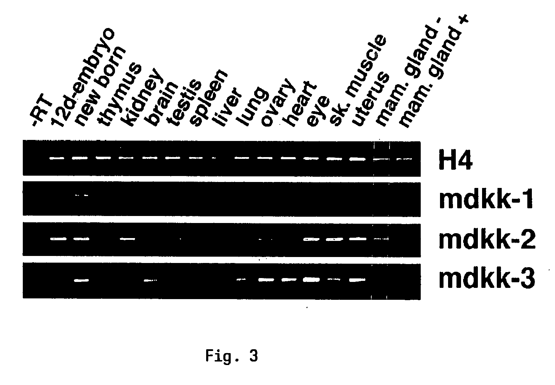

- Fig. 3: zeigt die Expression von drei (wnt-I) kodierenden DNAs, DKK-1, DKK-2 und DKK-3, in Geweben.

Die vorliegende Erfindung wird durch die nachstehenden Beispiele erläutert.

### Beispiel 1: Herstellung und Reinigung eines erfindungsgemäßen (wnt-I)

Zur Herstellung eines erfindungsgemäßen (wtn-I) wurde die DNA von Fig. 2.6, phdkk-1 mit Bam HI-Linkern versehen, anschließend mit Bam HI gespalten und in den mit Bam HI gespaltenen Expressionsvektors pQE-8 (Qiagen) inseriert. Es wurde das Expressionsplasmid pQ/wnt-1 erhalten. Ein solches kodiert für ein Fusionsprotein aus 6 Histidin-Resten (N-Terminuspartner) und einem erfindungsgemäßen (wnt-I) (C-Terminuspartner). pQ/wnt-I wurde zur Transformation von E.coli SG 13009(vgl. Gottesman, S. et al., J. Bacteriol. 148, (1981), 265-273) verwendet. Die Bakterien wurden in einem LB-Medium mit 100µg/ml Ampicillin und 25µg/ml Kanamycin kultiviert und 4 h mit 60µM Isopropyl-β-D-Thiogalactopyranosid (IPTG) induziert. Durch Zugabe von 6 M Guanidinhydrochlorid wurde eine Lyse der Bakterien erreicht, anschließend wurde mit dem Lysat eine Chromatographie (Ni-NTA-Resin) in Gegenwart von 8 M Harnstoff entsprechend der Angaben des Herstellers (Diagen) des Chromatographie-Materials durchgeführt. Das gebundene Fusionsprotein wurde in einem Puffer mit pH 3,5 eluiert. Nach seiner Neutralisierung wurde das Fusionsprotein einer 18 % SDS-Polyacrylamid-Gelelektrophorese unterworfen und mit Coomassie-Blau angefärbt (vgl. Thomas, J.O. und Kornberg, R.D., J.Mol.Biol. 149 (1975), 709-733).

Es zeigte sich, daß ein erfindungsgemäßes (Fusions)protein in hochreiner Form hergestellt werden kann.

### Beispiel 2: Herstellung und Nachweis eines erfindungsgemäßen Antikörpers

Ein erfindungsgemäßes Fusionsprotein von Beispiel 1 wurde einer 18 % SDS-Polyacrylamid-Gelelektrophorese unterzogen. Nach Anfärbung des Gels mit 4 M Natriumacetat wurde eine ca. 40 kD Bande aus dem Gel herausgeschnitten und in Phosphat gepufferter Kochsalzlösung inkubiert. Gel-Stücke wurden sedimentiert, bevor die Proteinkonzentration des Überstandes durch eine SDS-Polyacrylamid-Gelelektrophorese, der eine Coomassie-Blau-Färbung folgte, bestimmt wurde. Mit dem Gel-gereinigten Fusionsprotein wurden Tiere wie folgt immunisiert:

### Immunisierungsprotokoll für polyklonale Antikörper im Kaninchen

Pro Immunisierung wurden 35µg Gel-gereinigtes Fusionsprotein in 0,7 ml PBS und 0,7 ml komplettem bzw. inkomplettem Freund's Adjuvans eingesetzt.
- Tag 0:: 1. Immunisierung (komplettes Freund's Adjuvans)
- Tag 14:: 2. Immunisierung (inkomplettes Freund's Adjuvans; icFA)
- Tag 28:: 3. Immunisierung (icFA)
- Tag 56:: 4. Immunisierung (icFA)
- Tag 80:: Ausbluten

Das Serum des Kaninchens wurde im Immunoblot getestet. Hierzu wurde ein erfindungsgemäßes Fusionsprotein von Beispiel 1 einer SDS-Polyacrylamid-Gelelektrophorese unterzogen und auf ein Nitrocellulosefilter übertragen (vgl. Khyse-Andersen, J., J. Biochem. Biophys. Meth. 10, (1984), 203-209). Die Western Blot-Analyse wurde wie in Bock, C.-T. et al., Virus Genes 8, (1994), 215-229, beschrieben, durchgeführt. Hierzu wurde das Nitrocellulosefilter eine Stunde bei 37°C mit einem ersten Antikörper inkubiert. Dieser Antikörper war das Serum des Kaninchens (1:10000 in PBS). Nach mehreren Waschschritten mit PBS wurde das Nitrocellulosefilter mit einem zweiten Antikörper inkubiert. Dieser Antikörper war ein mit alkalischer Phosphatase gekoppelter monoklonaler Ziege Anti-Kaninchen-lgG-Antikörper (Dianova) (1:5000) in PBS. Nach 30-minütiger Inkubation bei 37°C folgten mehrere Waschschritte mit PBS und anschließend die alkalische Phosphatase-Nachweisreaktion mit Entwicklerlösung (36µM 5' Bromo-4-chloro-3-indolylphosphat, 400µM Nitroblau-tetrazolium, 100mM Tris-HCI, pH 9.5, 100 mM NaCl, 5 mM MgCl₂) bei Raumtemperatur, bis Banden sichtbar waren.

Es zeigte sich, daß erfindungsgemäße, polyklonale Antikörper hergestellt werden können.

### Immunisierungsprotokoll für polyklonale Antikörper im Huhn

Pro Immunisierung wurden 40µg Gel-gereinigtes Fusionsprotein in 0,8 ml PBS und 0,8 ml komplettem bzw. inkomplettem Freund's Adjuvans eingesetzt.
- Tag O.: 1. Immunisierung (komplettes Freund's Adjuvans)
- Tag 28:: 2. Immunisierung (inkomplettes Freund's Adjuvans; icFA)
- Tag 50:: 3. Immunisierung (icFA)

Aus Eigelb wurden Antikörper extrahiert und im Western Blot getestet. Es wurden erfindungsgemäße, polyklonale Antikörper nachgewiesen.

### Immunisierungsprotokoll für monoklonale Antikörper der Maus

Pro Immunisierung wurden 12µg Gel-gereinigtes Fusionsprotein in 0,25 ml PBS und 0,25 ml komplettem bzw. inkomplettem Freund's Adjuvans eingesetzt; bei der 4. Immunisierung war das Fusionsprotein in 0,5 ml (ohne Adjuvans) gelöst.
- Tag O.: 1. Immunisierung (komplettes Freund's Adjuvans)
- Tag 28:: 2. Immunisierung (inkomplettes Freund's Adjuvans; icFA)
- Tag 56:: 3. Immunisierung (icFA)
- Tag 84:: 4. Immunisierung (PBS)
- Tag 87:: Fusion

Überstände von Hybridomen wurden im Western Blot getestet. Erfindungsgemäße, monoklonale Antikörper wurden nachgewiesen.

**Tabelle 1: Expression von erfindungsgemäßen DNAs in Mausembryonen**

| | **Dkk-1** | **Dkk-2** | **Dkk-3** |
|---|---|---|---|
| **Neuroepithelium** | | | |
| E9.5 diencephalon | +++ ventral | +++ medial | + medial |
| E12.5 | telencephalon M/mantle | hypothatamus | telencephalon M/ ventricular zone |
| Eye | pigmented epithelium | chornid | retina |
| Spinal cord | -/+ | - | ventricular zone Roof plate |
| **Mesoderm:** | | | |
| Heart E10 | bulbis cordis Endocardium septum transversum | endothelium | myocardium |
| Heart E12 | endocardial cushion | endothelium | endocardial cushion |
| Blood vessels | +++ aorta | +++ pulmonary artery | +++ aorta + pulmonary ailery |
| Limbbud mesenchyme | E9 S | I | D |
| Bone E12 | perichondrium | S/mesenchyme | perichondrium I/mesenchyme |
| Bone E15 | Ossification centers | | - |
| Urogenital | nephric duct S-shaped body Comma shaped body | metanephric mesenchyme | - |
| Palate | +++ | ++ | + |
| Hair follicle | +++ mesenchyme + epithelium | + - | + - |
| Tooth mesenchyme | - | - | +++ |
| Trunk mesoderm | +/- | +++ | ++ |

| | | | |
|---|---|---|---|
| Legende: Mesoderm: (D) deep. (I) intermediate (L) lateral, (M) medial, (S), superficial. Expressionshöhe: (-) absence, (+/-) very weak expression, (+) medium, (++) strong, (+++) very strong. | | | |

## Patentansprüche

1. Isoliertes Inhibitor-Protein des wnt-Signalwegs, wobei das Inhibitor-Protein mindestens eine aus Konsensus-Sequenz I oder Konsensus-Sequenz II, wie in der Figur 1 angegeben, umfasst, wobei die Zahl der variablen Aminosäuren, die durch die Sterne in der Konsensus-Sequenz I oder Konsensus-Sequenz II angegeben sind, mit der Zahl der Aminosäuren in der entsprechenden Region eines dkk-Polypeptids identisch ist.

2. Inhibitor-Protein nach Anspruch 1, wobei das dkk-Polypeptid aus einem Polypeptid ausgewählt ist, das durch eine DNA, die aus mindestens einem aus SEQ ID NO:1 bis SEQ ID NO:7 ausgewählt ist, oder durch eine aufgrund der Degeneration des genetischen Kodes verwandte DNA kodiert ist.

3. Inhibitor-Protein nach Anspruch 2, wobei das dkk-Polypeptid ein Säugetier-dkk1-Polypeptid ist, das durch eine DNA, die aus SEQ ID NO:3 und SEQ ID NO:5 ausgewählt ist, oder durch eine aufgrund der Degeneration des genetischen Kodes verwandte DNA kodiert ist.

4. Isoliertes Nukleotid, das für das Inhibitor-Protein nach Anspruch 2 kodiert.

5. Isolierte DNA, die ein Inhibitor-Protein des wnt-Signalwegs kodiert, wobei die DNA aus SEQ ID NO:1, SEQ ID NO:2, SEQ ID NO:3, SEQ ID NO:4, SEQ ID NO:5, SEQ ID NO:6 oder SEQ ID NO:7 oder einer DNA ausgewählt ist, die aufgrund der Degeneration des genetischen Kodes mit dieser DNA verwandt ist.

6. Expressionsplasmid, umfassend die DNA nach Anspruch 5.

7. Isolierte Zelle, enthaltend das Expressionsplasmid nach Anspruch 6.

8. Verfahren zur Herstellung eines wnt-Signalweg-Inhibitor-Proteins, umfassend das Kultivieren der isolierten Zelle gemäß Anspruch 7 unter geeigneten Bedingungen.

9. Isolierte DNA nach Anspruch 5, wobei die DNA SEQ ID NO:3 ist.

10. Isolierte DNA nach Anspruch 5, wobei die DNA SEQ ID NO:5 ist.

11. Isolierter Antikörper, der gegen ein wnt-Signalweg-Inhibitor-Protein gerichtet ist, wobei das Inhibitor-Protein mindestens eine aus Konsensus-Sequenz I und Konsensus-Sequenz II, wie in der Figur 1 angegeben, umfasst, wobei die Zahl der variablen Aminosäuren, die durch die Sterne in der Konsensus-Sequenz I oder Konsensus-Sequenz II angegeben sind, mit der Zahl der Aminosäuren in der entsprechenden Region eines Polypeptids identisch ist, das durch eine DNA, die aus mindestens einer aus SEQ ID NO:1 bis SEQ ID NO: 7 ausgewählt ist, oder durch ein Polynukleotid kodiert wird, das mit der DNA im Hinblick auf den degenerierten genetischen Kode verwandt ist.

12. Antikörper nach Anspruch 11, wobei das wnt-Signalweg-Inhibitor-Protein die Konsensus-Sequenz I umfasst und die Zahl der variablen Aminosäuren mit dem Polypeptid identisch ist, die durch ein Säugetier-dkk1 kodiert wird.

13. Antikörper nach Anspruch 11, wobei das wnt-Signalweg-Inhibitor-Protein die Konsensus-Sequenz I umfasst und die Zahl der variablen Aminosäuren mit dem durch ein humanes dkk1 kodiertes Polypeptid identisch ist.

14. Antikörper nach Anspruch 11, wobei das wnt-Signalweg-Inhibitor-Protein die Konsensus-Sequenz I umfasst und die Zahl der variablen Aminosäuren mit dem Polypeptid identisch ist, das durch das Polynukleotid in SEQ ID NO:3 oder durch ein Polynukleotid kodiert ist, das mit der DNA im Hinblick auf den degenerierten genetischen Kode verwandt ist.

15. Antikörper nach Anspruch 11, wobei das wnt-Signalweg-Inhibitor-Protein die Konsensus-Sequenz I umfasst und die Zahl der variablen Aminosäuren mit dem Polypeptid identisch ist, das durch das Polynukleotid in SEQ ID NO:5 oder durch ein Polynukleotid kodiert ist, das mit der DNA im Hinblick auf den degenerierten genetischen Kode verwandt ist.

16. Antikörper nach Anspruch 11, wobei das wnt-Signalweg-Inhibitor-Protein die Konsensus-Sequenz II umfasst und die Zahl der variablen Aminosäuren mit der Zahl im durch ein Säugetier-dkk1 kodierten Polypeptid identisch ist.

17. Antikörper nach Anspruch 11, wobei das wnt-Signalweg-Inhibitor-Protein die Konsensus-Sequenz II umfasst, und die Zahl der variablen Aminosäuren mit der Zahl im durch ein humanes dkk1 kodierten Polypeptid identisch ist.

18. Antikörper nach Anspruch 11, wobei das wnt-Signalweg-Inhibitor-Protein die Konsensus-Sequenz II umfasst und die Zahl der variablen Aminosäuren mit den Polypeptiden identisch ist, die durch die Polynukleotide von SEQ ID NO:3 und SEQ ID NO:5 oder durch ein Polynukleotid kodiert sind, das mit der DNA im Hinblick auf den degenerierten genetischen Kode verwandt ist.

19. Antikörper nach Anspruch 11, wobei das wnt-Signalweg-Inhibitor-Protein die Konsensus-Sequenz II umfasst und die Zahl der variablen Aminosäuren mit einem Polypeptid identisch ist, das durch ein Polynukleotid, das aus SEQ ID NO:3 und SEQ ID NO:5 ausgewählt ist, oder durch ein Polynukleotid kodiert wird, das mit der DNA im Hinblick auf den degenerierten genetischen Kode verwandt ist.

20. Antikörper nach Anspruch 11, wobei das Polypeptid durch SEQ ID NO:3 oder durch ein Polynukleotid, das mit der DNA im Hinblick auf den degenerierten genetischen Kode verwandt ist, kodiert ist.

21. Antikörper nach Anspruch 11, wobei das Polypeptid durch SEQ ID NO:5 oder durch ein Polynukleotid, das mit der DNA im Hinblick auf den degenerierten genetischen Kode verwandt ist, kodiert wird.

22. Antikörper nach Anspruch 11, wobei der Antikörper ein polyklonaler Antikörper ist.

23. Antikörper nach Anspruch 11, wobei der Antikörper ein monoklonaler Antikörper ist.

24. Antikörper nach Anspruch 11, wobei das wnt-Signal-Inhibitor-Protein ein Teil eines Fusionsproteins ist.

25. Verwendung eines Antikörpers, der ein wnt-Signalweg-Inhibitor-Protein bindet zur Herstellung eines Medikaments zur Behandlung einer Erkrankung durch Hemmen eines wnt-Signalwegs, wobei das Inhibitor-Protein mindestens eines aus Konsensus-Sequenz I oder Konsensus-Sequenz II, wie in der Figur 1 angegeben, umfasst, wobei die Zahl der variablen Aminosäuren, die durch Sterne in der Consensus-Sequenz I oder der Consensus-Sequenz II angegeben sind, mit der Zahl der Aminosäuren in der entsprechenden Region eines Polypeptids identisch ist, das durch eine DNA, die aus mindestens einer aus SEQ ID NO:1 bis SEQ ID NO:7 ausgewählt ist, oder durch eine aufgrund der Degeneration des genetischen Kodes verwandte DNA kodiert ist.

26. Verwendung nach Anspruch 25, wobei das Inhibitor-Protein mindestens eine aus Konsensus-Sequenz I oder Konsensus-Sequenz II, wie in der Figur 1 angegeben, umfasst, wobei die Zahl der variablen Aminosäuren, die durch die Sterne in der Konsensus-Sequenz I und Konsensus-Sequenz II angegeben sind, mit der Zahl der Aminosäuren in der entsprechenden Region eines Säugetier-dkk1-Polypeptids identisch ist, das durch eine DNA, die aus SEQ ID NO:3 und SEQ ID NO:5 ausgewählt ist, oder durch eine aufgrund der Degeneration des genetischen Kodes verwandte DNA kodiert ist.

27. Verwendung nach Anspruch 25, wobei der Antikörper aus einem der Ansprüche 11 bis 24 ausgewählt ist.

28. Verwendung nach Anspruch 25, wobei ein Medikament zur Behandlung einer Erkrankung des Auges, des Knochens oder einer Tumorerkrankung hergestellt wird.

29. Verwendung nach Anspruch 28, wobei die Tumorerkrankung aus Kolonkarzinom, Mammakarzinom, oder Melanom ausgewählt ist.

30. Verwendung eines Antikörpers, der ein wnt-Signalweg-Inhibitor-Protein bindet, zur Herstellung einer diagnostischen oder therapeutischen Zusammensetzung für das Eingreifen in einen wnt-Signalweg, wobei das Inhibitor-Protein mindestens eines aus der Konsensus-Sequenz I oder Konsensus-Sequenz II, wie in der Figur 1 angegeben, umfasst, wobei die Zahl der variablen Aminosäuren, die durch die Sterne in der Konsensus-Sequenz I oder Konsensus-Sequenz II angegeben sind, mit der Zahl der Aminosäuren in der entsprechenden Region eines dkk1-Polypeptids identisch ist, das durch eine DNA, die aus SEQ ID NO:3 und SEQ ID NO:5 ausgewählt ist, oder durch eine aufgrund der Degeneration des genetischen Kodes verwandten DNA kodiert ist.

31. Verwendung nach Anspruch 30, wobei der Antikörper aus einem der Ansprüche 11 bis 24 ausgewählt ist.

32. Verwendung nach Anspruch 30, wobei die Zusammensetzung für die Behandlung von Erkrankungen des Auges, Erkrankungen des Knochens oder tumoralen Erkrankungen hergestellt wird.

33. Verwendung nach Anspruch 32, wobei die tumoralen Erkrankungen Kolonkarzinom, Mammakarzinom oder Melanom sind.

34. Verfahren zum Nachweis eines wnt-Signalweg-Inhibitor-Proteins in einer Probe, umfassend die Schritte:
Zugabe eines Antikörpers, der ein wnt-Signalweg-Inhibitor-Protein bindet, zu einer Probe und Nachweis des wnt-Signalweg-Inhibitor-Proteins mittels eines Western Blots, einem ELISA, einer Immunpräzipitation oder Immunfluoreszenz,
wobei das Inhibitor-Protein mindestens eine aus Konsensus-Sequenz I oder Konsensus-Sequenz II, wie in der Figur 1 angegeben ist, umfasst, wobei die Zahl der variablen Aminosäuren, die durch Sterne in der Konsensus-Sequenz I oder Konsensus-Sequenz II angegeben sind, mit der Zahl der Aminosäuren in der entsprechenden Region eines dkk1-Polypeptids identisch ist, das durch eine DNA, die aus SEQ ID NO:3 und SEQ ID NO:5 ausgewählt wird, oder durch eine aufgrund der Degeneration des genetischen Kodes verwandte DNA kodiert ist.

35. Verfahren nach Anspruch 34, wobei der Antikörper aus einem der Ansprüche 11 bis 24 ausgewählt ist.
